# EUROPEAN PATENT APPLICATION

(11) **EP 4 772 681 A1**
(43) Date of publication of application: **08.07.2026**
(21) Application number: 24866597.8
(22) Date of filing: 21.02.2024
(51) Int. Cl.: C40B 50/06, C12Q 1/6869

(54) **PREPARATION METHOD FOR SPATIAL BARCODE CHIP AND APPLICATION OF SPATIAL BARCODE CHIP**

(30) Priority: 13.10.2023 CN 202311323952
(71) Applicant: Beijing SeekGene Biosciences Co., Ltd., Beijing 102200 (CN)
(72) Inventor: HAN, Jinhuan, Beijing 102200 (CN); GUAN, Ying, Beijing 102200 (CN); FENG, Shijie, Beijing 102200 (CN); ZHI, Xiaoling, Beijing 102200 (CN); ZHI, Min, Beijing 102200 (CN); SANG, Guoqin, Beijing 102200 (CN); SHI, Huanhuan, Beijing 102200 (CN); WEI, Qingquan, Beijing 102200 (CN); JIAO, Shaozhuo, Beijing 102200 (CN); LI, Zongwen, Beijing 102200 (CN)
(74) Representative: Dantz, Jan Henning
(86) International application number: PCT/CN2024/077954
(87) International publication number: WO 2025/077082

(57) **Abstract**

The present disclosure discloses a method for preparing a spatial barcode chip and application thereof, including steps of: S10: providing a chip; S20: binding multiple nucleic acid molecules from a nucleic acid molecule library to cleavable sequences of the chip, respectively, and performing amplification to obtain multiple nucleic acid molecule clusters, where each of the nucleic acid molecules includes a spatial tag sequence and a library construction binding sequence, and a sequencing primer binding site for decoding between the spatial tag sequence and the library construction binding sequence, the spatial tag sequence of each nucleic acid molecule is different, and the nucleic acid molecule further includes a chip binding sequence which binds to the corresponding cleavable sequence on the chip; and S30: sequencing and decoding the nucleic acid molecule clusters to obtain the spatial barcode chip. The chip releases nucleic acid molecules onto single-cell nuclei, which are then captured as a whole by capture sequences, allowing for accurate and complete reading of the spatial information and genetic information of single cells.

## Description

### CROSS-REFERENCE TO RELATED APPLICATIONS

This application claims priority to Chinese Patent Application No. 202311323952.2, filed on October 13, 2023, which is incorporated herein by reference in its entirety.

### TECHNICAL FIELD

The present disclosure relates to the technical field of gene sequencing and tissue cell sample analysis, and in particular to a method for preparing a spatial barcode chip and its application.

### BACKGROUND

With the continuous development of single-cell sequencing technology, the composition and transcription levels of single cells can be detected through various techniques, greatly enhancing researchers' ability to analyze cell type-specific gene regulatory networks from heterogeneous samples. However, spatial information could not be detected by using existing techniques. Since spatial heterogeneity is a key feature of organ function, the spatial information of cells is crucial for studying cell fate regulation mechanisms and cell lineage development processes. Therefore, to better understand cells, it is necessary to simultaneously record their transcriptional heterogeneity and spatial coordinates.

In the prior art, to deeply understand nucleic acid information at the tissue level, scientists have developed various spatial omics technologies, mainly divided into two categories based on throughput. Low-throughput spatial omics technologies include in situ hybridization techniques (SeqFish and osm Fish) and in situ sequencing techniques (FISSEQ and STARmap). These techniques rely on probes designed for genes of interest, maintaining single-cell (even subcellular) resolution in each field of view. However, this method is usually limited by long image acquisition time and complex instrument requirements. High-throughput spatial omics technologies involve in situ capture based on spatial barcode technology, including 10× Visium, Slide-seq v2, Stereo-seq, and Seq-scope. These are based on sequences with spatial information on spatial chips hybridizing with RNA released by cells. The chips perform in situ reverse transcription to synthesize cDNA, collect cDNA, and finally construct sequencing libraries. The drawback is that RNA information is analyzed at the tissue position level, and then software is used to simulate the cell to which each RNA belongs based on its spatial information, rather than directly analyzing the actual physical tissue single cells. RNA diffusion or the presence of multiple cell layers can lead to cell localization distortion. Among them, the chips used by Stereo-seq and Seq-scope are spatial barcode chips obtained through sequencing, but the production methods are complicated and costly. After sequencing, the hybrid strand of the sequencing strand and template strand need to be processed to expose the captured sequence. Stereo-seq is based on Beijing Genomics Institution's (BGI) DNA nanoball (DNB) technology, in which DNBs are loaded onto array chips for sequencing and decoding; and after sequencing, the captured sequence is connected using the hybridized state of the sequencing and template strands. Seq-scope involves loading libraries synthesized with different spatial information onto an Illumina sequencing chip for sequencing and decoding; and after sequencing, restriction enzymes are used to cut the hybrid strand of the sequencing strand and template strand, thereby exposing the captured sequence. Therefore, there is an urgent need to develop a spatial barcode chip that is easy to prepare and inexpensive, and can accurately and completely detect the spatial information of single cells in tissues and the nucleic acid information of single cells.

### SUMMARY

A main objective of the present disclosure is to provide a method for preparing a spatial barcode chip and an application thereof. The present disclosure aims to solve the technical problems of high cost and complex methods in the preparation of spatial barcode chips in the prior art, and their inability to accurately and completely detect the spatial information of single cells and the nucleic acid information of single cells.

To achieve the above objective, the present disclosure provides a method for preparing a spatial barcode chip, including steps of:
S10: providing a chip;
S20: binding multiple nucleic acid molecules from a nucleic acid molecule library to cleavable sequences of the chip, respectively, and performing amplification to obtain multiple nucleic acid molecule clusters, where each of the nucleic acid molecules includes a spatial tag sequence and a library construction binding sequence, and a sequencing primer binding site for decoding between the spatial tag sequence and the library construction binding sequence, the spatial tag sequence of each nucleic acid molecule is different, and the nucleic acid molecule further includes a chip binding sequence which binds to the corresponding cleavable sequence on the chip; and
S30: sequencing and decoding the nucleic acid molecule clusters to obtain the spatial barcode chip.

Optionally, the library construction binding sequence includes a combination of one or more bases; and/or
the cleavable sequence of the chip includes any one of a disulfide-modified sequence, a dU-modified sequence, a dl-modified sequence, an AP site-modified sequence, an i8oxodG-modified sequence, a photolabile group-modified sequence, and a restriction enzyme recognition sequence.

Optionally, the chip is ligated to a primer for in situ amplification and the primer contains a cleavable sequence.

Optionally, the primer further contains a modified group with a ligation function and a dNTP derivative that provides dNTP in the amplification and has a chemical ligation function.

Optionally, in step S20, the nucleic acid molecule library is prepared through the following steps:
S201: providing various single-stranded nucleic acid molecules; and
S202: subjecting the various single-stranded nucleic acid molecules to a ligation reaction to obtain the nucleic acid molecule library.

Optionally, in step S201, the various single-stranded nucleic acid molecules include a first nucleic acid molecule, a second nucleic acid molecule, and a third nucleic acid molecule;
where a sequence of the first nucleic acid molecule is shown as SEQ ID NO.1;
a sequence of the second nucleic acid molecule is shown as SEQ ID NO.2; and/or
a sequence of the third nucleic acid molecule is shown as SEQ ID NO.3.

Optionally, in step S202, a sequence of the nucleic acid molecule is shown as SEQ ID NO.4.

Optionally, the library construction binding sequence is 5 bp to 100 bp; and/or a density of each of the nucleic acid molecule clusters is 0.1 um² to 2 um²; and/or the spatial tag sequence is 15 bp to 40 bp.

The present disclosure provides a high-throughput single-cell library construction method, including the following steps:
S40: providing a spatial barcode chip prepared by any one of the above methods for preparing a spatial barcode chip;
S50: contacting the spatial barcode chip with a slice of a tissue to be tested and performing dissociation to prepare a single-cell nuclei suspension;
S60: constructing a library for the single-cell nuclei suspension using a high-throughput single-cell library construction and sequencing platform to obtain a transcriptome library and a spatial tag library; and
S70: sequencing the transcriptome library and the spatial tag library to obtain single-cell library data containing spatial information of cells in tissue.

Optionally, step S50 includes the following step:
S501: contacting the spatial barcode chip with the slice of the tissue to be tested, then baking the slice, extracting nuclei, releasing nucleic acid molecules with spatial information, and performing fixation and dissociation to obtain the single-cell nuclei suspension.

Optionally, a reagent for extracting nuclei in step S501 includes at least one of NP40, Chaps, Tween20, TritonX-100, SDS, and deoxycholic acid; and/or a reagent for fixation includes at least one of an aldehyde fixative solution, an acid fixative solution, an alcohol fixative solution, and a DSP crosslinker.

The present disclosure further provides a sequencing method including the following steps:
constructing a single-cell library using the high-throughput single-cell library construction method as described in any one of the above embodiments to obtain a transcriptome library and a spatial tag library;
sequencing the transcriptome library and the spatial tag library to obtain single-cell transcriptome data and spatial tag library data; and
mapping spatial information and genetic information of nucleic acid molecules to the tissue to be tested to obtain spatial information and genetic information of single cells in the tissue to be tested.

In the present disclosure, the nucleic acid molecules include a chip binding sequence that binds to a cleavable sequence on the chip, and after amplification, sequencing, and decoding on the chip, the spatial barcode chip is obtained. The sequencing primer binding site for decoding in the nucleic acid molecule is positioned between the spatial tag sequence and the library construction binding sequence. By processing the cleavable sequence on the spatial barcode chip, the nucleic acid molecules can be released from the chip surface and subsequently enter into the cell nuclei where they are fixed. The nucleic acid molecules have a library construction binding sequence, allowing the spatial tag sequences to be captured as a whole with the single-cell nuclei during subsequent library construction. After sequencing, the spatial information and genetic information of single cells can be accurately and completely read.

### BRIEF DESCRIPTION OF THE DRAWINGS

To more clearly illustrate the technical solutions in the embodiments of the present disclosure or the prior art, a brief introduction to the accompanying drawings required for the description of the embodiments or the prior art is provided below. It is evident that the accompanying drawings described below are merely some embodiments of the present disclosure. For a person of ordinary skill in the art, other drawings can be obtained based on the structures shown in these drawings without creative efforts.
FIG. 1 is a schematic diagram of the principle for constructing a spatial chip containing spatial information according to an embodiment of the present disclosure;
FIG. 2 is a schematic diagram of the principle of single-cell sequencing with spatial information according to an embodiment of the present disclosure;
FIG. 3 is a schematic structural diagram of a nucleic acid molecule structure with spatial information on a nucleic acid chip according to an embodiment of the present disclosure;
FIG. 4 is a schematic diagram of the principle for constructing a nucleic acid molecule library according to an embodiment of the present disclosure;
FIG. 5 is a structural diagram of a single-cell transcriptome library according to an embodiment of the present disclosure;
FIG. 6 is a structural diagram of a spatial tag library according to an embodiment of the present disclosure;
FIG. 7 is a diagram of quality control of a single-cell transcriptome library according to an embodiment of the present disclosure; and
FIG. 8 is a diagram of quality control of a spatial tag library according to an embodiment of the present disclosure.

The realization, functional characteristics, and advantages of the present disclosure will be further explained in conjunction with the embodiments and with reference to the accompanying drawings.

### DETAILED DESCRIPTION

To make the objectives, technical solutions, and advantages of embodiments of the present disclosure clearer, the technical solutions in the embodiments of the present disclosure will be described clearly and completely below. For an embodiment for which a detailed condition is not specified, the embodiment is conducted under a regular condition or a condition recommended by a manufacturer. Any reagent or instrument used for which no manufacturer is specified is a regular commercially available product. In addition, "and/or" in this specification includes three parallel schemes. For example, "A and/or B" includes a scheme A, or a scheme B, or both the scheme A and the scheme B. In addition, technical solutions in the embodiments may be combined with each other, provided that they can be implemented by a person of ordinary skill in the art. When the combination of technical solutions is contradictory or unimplementable, it should not be considered that the combination of technical solutions exists or falls within the claimed protection scope of the present disclosure. All other embodiments obtained by a person of ordinary skill in the art on the basis of the embodiments of the present disclosure without creative effort shall fall within the scope of protection of the present disclosure.

The chips used in Stereo-seq and Seq-scope are spatial barcode chips obtained through sequencing. However, the production methods are tedious and costly. After sequencing, the hybrid strand of the sequencing strand and the template strand needs to be processed to expose the capture sequence. Based on the hybridization of the sequence with spatial information on the spatial chip with RNA released by the cells, the reverse transcription and synthesis of cDNA is completed in situ on the chip, the cDNA is collected, and finally, a sequencing library is constructed. The disadvantages are that RNA information is analyzed at the tissue position level, and then software is used to simulate the cell to which each RNA belongs based on the spatial information of each RNA, rather than directly analyzing the tissue single cells at a true physical level. In addition, RNA diffusion or the presence of multiple cell layers can lead to cell localization distortion. Stereo-seq is based on BGI's DNA nanoball (DNB) technology, in which DNBs are loaded onto array chips for sequencing and decoding; and after sequencing, the captured sequence is connected using the hybridized state of the sequencing and template strands. Seq-scope involves loading libraries synthesized with different spatial information onto an Illumina sequencing chip for sequencing and decoding; and after sequencing, restriction enzymes are used to cut the hybrid strand of the sequencing strand and template strand, thereby exposing the captured sequence. Therefore, there is an urgent need to develop a spatial barcode chip that is easy to prepare and inexpensive, and can accurately and completely detect the spatial information of single cells in tissues and the nucleic acid information of single cells.

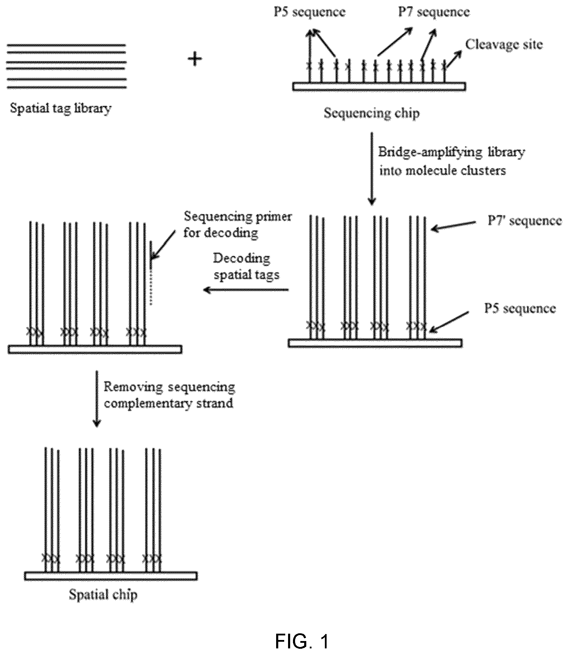

In view of this, the present disclosure provides a method for preparing a spatial barcode chip and application thereof. As shown in FIG. 1, the present disclosure is intended to provide a spatial barcode chip with a simple method and low cost, which can accurately and completely detect spatial information of single cells in the tissue and the nucleic acid information of the single cells.

An embodiment of a method for preparing a spatial barcode chip provided in the present disclosure includes the following steps:
S10. providing a chip;
S20. binding multiple nucleic acid molecules from a nucleic acid molecule library to cleavable sequences of the chip, respectively, and performing amplification to obtain multiple nucleic acid molecule clusters, where each of the nucleic acid molecules includes a spatial tag sequence and a library construction binding sequence, and a sequencing primer binding site for decoding between the spatial tag sequence and the library construction binding sequence, the spatial tag sequence of each nucleic acid molecule is different, and the nucleic acid molecule further includes a chip binding sequence which binds to the corresponding cleavable sequence on the chip; and
S30: sequencing and decoding the nucleic acid molecule clusters to obtain the spatial barcode chip.

In the present disclosure, the nucleic acid molecules include a chip binding sequence that binds to a cleavable sequence on the chip, and after amplification, sequencing, and decoding on the chip, the spatial barcode chip is obtained. The sequencing primer binding site for decoding in the nucleic acid molecule is positioned between the spatial tag sequence and the library construction binding sequence. By processing the cleavable sequence on the spatial barcode chip, the nucleic acid molecules can be released from the chip surface and subsequently enter into the cell nuclei where they are fixed. The nucleic acid molecules have a library construction binding sequence, allowing the spatial tag sequences to be captured as a whole with the single-cell nuclei during subsequent library construction. After sequencing, the spatial information and genetic information of single cells can be accurately and completely read.

Further, the library construction binding sequence includes one base or a combination of at least two bases.

It should be noted that the library construction binding sequence is any sequence that can be captured by the capture sequence, as long as the capture sequence is correspondingly set in the subsequent library construction process to capture the library construction binding sequence. For example, for a single-cell spatial 3' transcriptome, if the library construction binding sequence in the nucleic acid molecule is oligo dA, the oligo dT sequence can be used to capture the library construction binding sequence with a spatial tag and the mRNA, to obtain a spatial tag library and a transcriptome library. If the library construction binding sequence in the nucleic acid molecule is ACAGCAAA, the oligo dT sequence and the sequence recognizing ACAGCAAA are both used as capture sequences, the oligo dT sequence captures mRNA, and the sequence recognizing ACAGCAAA captures the library construction binding sequence with the spatial tag to obtain the transcriptome library and the spatial tag library. In addition, the library construction binding sequence can be captured by the capture sequence without being exposed by enzyme digestion, which not only reduces operations but also further lowers costs.

It should be noted that the cleavable sequence on the chip includes a first fixed sequence and a second fixed sequence, and the chip binding sequence in the nucleic acid molecule includes a chip binding sequence 1 and a chip binding sequence 2. The chip binding sequence 1 is identical to the first fixed sequence, the chip binding sequence 1 is at the 5 ' end of the nucleic acid molecule; the chip binding sequence 2 is complementary to the second fixed sequence, and the chip binding sequence 2 is at the 3 ' end of the nucleic acid molecule. Therefore, the nucleic acid molecule can be directly amplified onto the chip.

Further, the cleavable sequence of the chip includes any one of a disulfide-modified sequence, a dU-modified sequence, a dl-modified sequence, an AP site-modified sequence, an i8oxodG-modified sequence, a photolabile group-modified sequence, and a restriction enzyme recognition sequence, to recognize and thus accurately cleave the sequence.

In particular, in some embodiments, the cleavable sequence on the chip includes a first fixed sequence and a second fixed sequence. The first fixed sequence is a P5 sequence, and the P5 sequence includes any one of a disulfide-modified sequence, a dU-modified sequence, a dl-modified sequence, an AP site-modified sequence, an i8oxodG-modified sequence, a photolabile group-modified sequence, and a restriction enzyme recognition sequence. The second fixed sequence is a P7 sequence which includes any one of a disulfide-modified sequence, a dU-modified sequence, a dl-modified sequence, an AP site-modified sequence, an i8oxodG-modified sequence, a photolabile group-modified sequence, and a restriction enzyme recognition sequence. In addition, the first fixed sequence and the second fixed sequence have different modifications, to prevent simultaneous cleavage of both the first fixed sequence and the second fixed sequence.

It should be noted that by setting a sequencing primer binding site for decoding after the spatial tag sequence, the spatial tag sequence in the spatial sequencing chip can be read. A sequencing adapter sequence is used to amplify sample identification tags for different sequencing samples during subsequent library construction. The spatial tag sequence is a sequence that needs to be sequenced and identified in the present disclosure.

In some embodiments, the chip is ligated to a primer for in situ amplification, and the primer contains a cleavable sequence.

In some embodiments, the primer further contains a modified group with a ligation function (for example, an amino group, biotin, a carboxyl group, or the like), and a dNTP derivative that provides dNTP in amplification and has a chemical ligation function (for example, bases into which an amino modification can be incorporated, for example, 7-ap-7-Deaza-2'-dCTP, 5-ap-2'-dCTP and 7-ap-7-Deaza-2'-dGTP).

The method according to the present disclosure is based on a high-throughput sequencing process in which sequencing and synthesis are simultaneously performed, which can achieve the preparation of spatial chips without adding any extra steps. Compared with the SeqScope chip preparation scheme, the method reduces the restriction enzyme digestion steps; and compared with the chip preparation scheme used in stereo-seq, the method reduces the primer ligation steps, resulting in lower costs, better processes, and easier mass production. Additionally, the introduction of conditional cleavable sequences enables the construction of the spatial transcriptomic library at the true single-cell level.

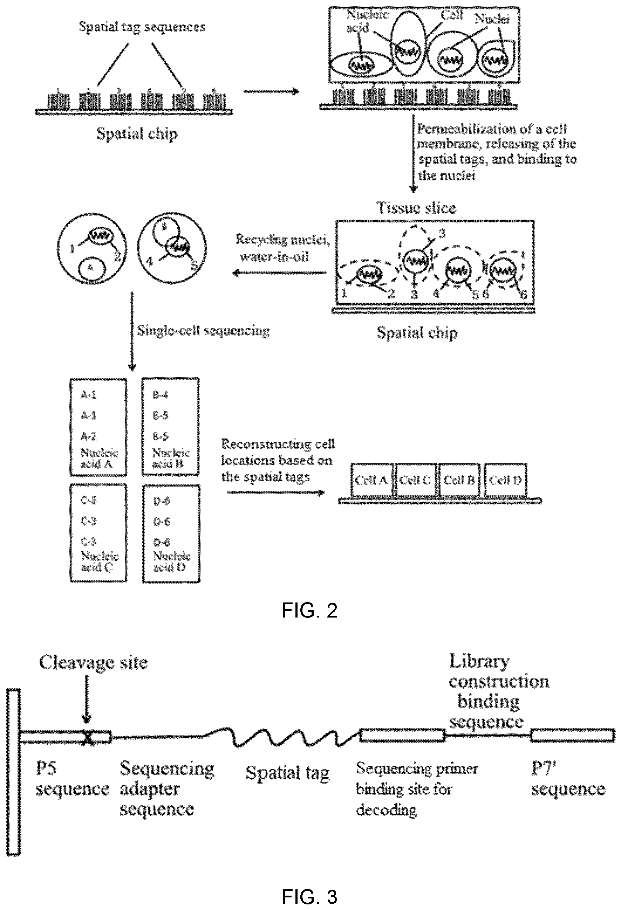

In particular, the operational steps in some embodiments are as follows: referring to the schematic diagrams of the principle for constructing a spatial chip and the principle of single-cell sequencing with spatial information as shown in FIG. 1 and FIG. 2, the steps include:
S10. providing a chip having a cleavable sequence, where the cleavable sequence on the chip is generally specific P5 or P7 sequence, the nucleic acid molecule library with different spatial tag sequences designed in advance in the present disclosure contains a chip binding sequence having a sequence identical to P5 and a sequence complementary to P7, i.e., a P5 sequence and a P7' sequence, and performing complementary strand extension using a sequence to be tested as a template, with the ends of the extended strand being P5' and P7, and therefore, the nucleic acid molecule library can be fixed on the chip through complementary hybridization;
S20. hybridizing the P5' sequence of the extended strand with the P5 sequence on the surface of the chip to synthesize the strand, forming a bridge structure, unwinding the synthesized double strand, and then allowing them to complementarily hybridize with other primers on the Flowcell, respectively, then extending, unwinding, hybridizing, extending, unwinding, and repeating the cycle, to generate millions of nucleic acid molecule clusters, cloning and amplifying all fragments, with the amplification here being bridge amplification, as shown in FIG. 1; each molecule cluster on the chip has spatial information encoded by a known nucleic acid base sequence, different molecule clusters have nucleic acid molecules with different base sequences, and the same molecule cluster has nucleic acid molecules with the same base sequence;
S30. after bridge amplification is completed, cutting off the P7 sequence on the chip, retaining the P5 sequence molecule clusters, reading the spatial tag sequence, the binding region of the sequencing primer being the sequencing primer binding site for decoding, then performing cyclic sequencing reactions on the sequencing chip, decoding the spatial tags, each cluster having spatial information encoded by a known nucleic acid base sequence, after sequencing is completed, performing NaOH denaturation, removing the sequencing extended strand, retaining only the original template strand of the spatial tag sequence molecules on the chip;
S40. using a laser to cut the chip, cutting the chip into a size of 5.5 mm*15 mm, one flowcell can be cut into 4 chips, with the side containing the nucleic acid sequence facing up, fixing the chip on a slide using transparent double-sided tape, cleaning the chip and sealing it in a chip box, storing it at 4°C for future use to obtain the spatial barcode chip.

Further, in step S20, the nucleic acid molecule library is prepared through the following steps:
S201: providing various single-stranded nucleic acid molecules; and
S202: subjecting the various single-stranded nucleic acid molecules to a ligation reaction to obtain the nucleic acid molecule library.

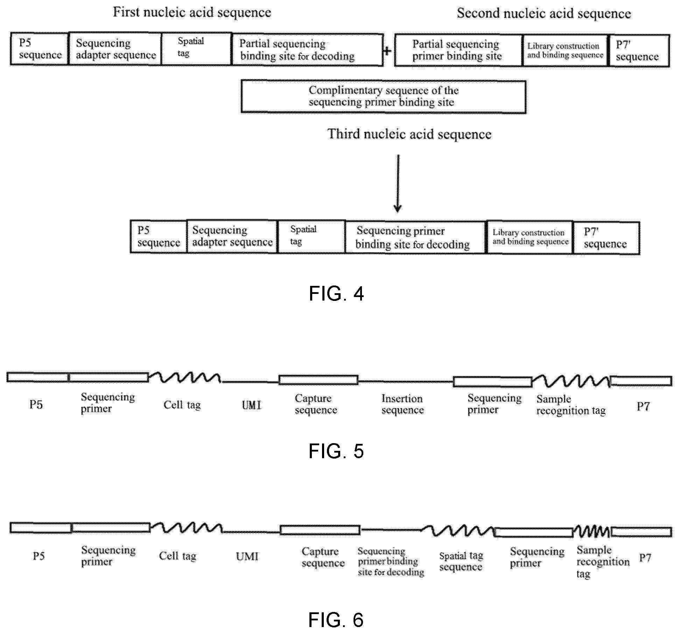

It should be noted that, referring to FIG. 3 and FIG. 4, FIG. 3 shows the structure of a nucleic acid molecule with a spatial tag sequence on the spatial chip, and the method shown in Figure 4 is used to connect them into a nucleic acid molecule library with spatial tag sequences, thereby constructing a nucleic acid molecule library with different spatial tag sequences.

Further, in step S201, the various single-stranded nucleic acid molecules include a first nucleic acid molecule, a second nucleic acid molecule, and a third nucleic acid molecule;
where a sequence of the first nucleic acid molecule is shown as SEQ ID NO.1: AATGATACGGCGACCACCGAGATCTACACGTGACTGGAGTTCAGACGTGTGCT CTTCCGATCTNNNNNNNNNNNNNNNNNNNNNNNNNNNNNNNNTCTTGTGACTA CAGCACC, where N base can be any one of ATCG bases.

A sequence of the second nucleic acid molecule is shown as SEQ ID NO.2: p-GACTTTCACCAGTCCATGATTCTC GTATGCCGTCTTCTGCTTG, where P is phosphate modification.

A sequence of the third nucleic acid molecule is shown as SEQ ID NO.3: GGTGAAAGTCGGTGCTGTAGT.

Further, different single-stranded nucleic acid molecules in Step S201 are synthesized into nucleic acid molecules with spatial tag sequences via DNA ligation reaction. A sequence of the nucleic acid molecule is shown as SEQ ID NO.4:

AATGATACGGCGACCACCGAGATCTACACGTGACTGGAGTTCAGACGT
GTGCTCTTCCGATCTNNNNNNNNNNNNNNNNNNNNNNNNNNNNNNNNTCTTGT
GACTACAGCACCGACTTTCACCAGTCCATGATAAAAAAAAAAAAAAAAAAAAAA
AAAAAAAAATCTCGTATGCCGTCTTCTGCTTG

In particular, in some embodiments, the method shown in FIG. 4 is used to connect them into a nucleic acid molecule library with spatial tag sequences, and the synthesis result is as shown in the figure.

1. The first nucleic acid sequence, the second nucleic acid sequence and the third nucleic acid sequence are synthesized, and then based on the DNA ligation reaction, the first nucleic acid sequence structure includes a P5 sequence, a sequencing adapter sequence, a spatial tag sequence and a partial sequencing primer binding site for decoding sequence in the order from 5' to 3', and the second nucleic acid sequence structure includes a partial sequencing primer binding site for decoding sequence, a library construction binding sequence, and a P7' sequence in the order from 5' to 3'. Based on the DNA ligation reaction, the 5' end of the second nucleic acid sequence has a phosphate modification, and the third nucleic acid sequence is a complementary sequence to the partial sequencing primer binding site.

Further, the library construction binding sequence is 5 bp to 100 bp, and preferably, can be 5 bp to 40 bp, and the capture effect is better within this range.

Further, in the nucleic acid molecule clusters, a density of each nucleic acid molecule cluster is 0.1 µm² to 2 µm², and in particular, can be 0.2 µm² to 1.5 µm², or preferably, 0.5 µm² to 1 µm². When the nucleic acid molecule cluster is within the range, not only sequencing and decoding resolution is high, but also more nucleic acid molecules can be cleaved.

The spatial tag sequence is 15 bp to 40 bp.

The present disclosure provides a high-throughput single-cell library construction method, including the following steps:
S40: providing a spatial barcode chip prepared by any one of the above methods for preparing a spatial barcode chip;
S50: contacting the spatial barcode chip with a slice of a tissue to be tested and performing dissociation to prepare a single-cell nuclei suspension;
S60: constructing a library for the single-cell nuclei suspension using a high-throughput single-cell library construction and sequencing platform to obtain a transcriptome library and a spatial tag library; and
S70: sequencing the transcriptome library and the spatial tag library to obtain single-cell library data containing spatial information of cells in tissue.

Further, step S50 includes the following steps: contacting the spatial barcode chip with the slice of the tissue to be tested, then baking the slice, extracting nuclei, releasing nucleic acid molecules with spatial information, and performing fixation and dissociation to obtain the single-cell nuclei suspension.

Further, a reagent for extracting nuclei in step S501 includes any one of NP40, Chaps, Tween20, TritonX-100, SDS, and deoxycholic acid, to damage an intact cell membrane.

A reagent for fixation includes any one of an aldehyde fixative solution, an acid fixative solution, an alcohol fixative solution, and a DSP crosslinker.

In particular, in some embodiments, the high-throughput single-cell library construction steps include:
contacting a slice of a tissue to be tested with the spatial barcode chip with spatial information, where a thickness of the slice of the tissue to be tested is 10 µm to 50 µm; and then baking the slice of the tissue, extracting nuclei, releasing the nucleic acid molecule with spatial information, and performing fixation and dissociation to obtain the single-cell nuclei suspension, the tissue slice being baked at 37°C for 1 minute to 5 minutes, the nuclei extraction process including disrupting the intact cell membrane by using a nuclei extraction reagent at a specific concentration, where the nuclei extraction reagent includes NP40, Chaps, Tween20, TritonX-100, SDS, or deoxycholic acid, and preferably, NP40 or Chaps; then using User enzyme to cleave the dU-modified bases on the P5 sequence to release nucleic acid molecules, the nucleic acid molecules with spatial information being transferred into the nuclei of the tissue slice by diffusion, removing unbound nucleic acid molecules; where the fixative reagent also needs to be added with Qubit ssDNA dye to stain the slice, after staining, taking fluorescence photos, the spatial location of the nuclei being marked through ssDNA staining, then recovering and dissociating the tissue slice to prepare a single-cell nuclei suspension, ensuring that the binding relationship between the nucleic acid with the spatial information and the cells is not disrupted during the recovery process.

In particular, in some embodiments, the high-throughput single-cell library construction and sequencing platform is used to construct the library for the single-cell nuclei suspension. Mature 3' library construction kits, such as the SeekOne^{®} 3' Single Cell Transcriptome reagent experimental procedure solution, can be used to obtain the spatial tag library and the single-cell 3' transcriptome library. Alternatively, library construction kits with capture sequences can be prepared based on the library binding sequences, the capture sequences can capture intracellular nucleic acid sequences as well as library binding sequences, and then the single-cell library and the spatial tag library are obtained, respectively. The following sequencing steps are performed using the library construction kits containing the capture sequences.

The present disclosure further provides a sequencing method including the following steps:
constructing a single-cell library using the method for constructing a single-cell library containing spatial information of cells in tissue to obtain a transcriptome library and a spatial tag library;
sequencing the obtained transcriptome library and the spatial tag library to obtain single-cell transcriptome data and spatial tag library data; and
mapping spatial information and genetic information of nucleic acid molecules to the tissue to be tested to obtain spatial information and genetic information of single cells in the tissue to be tested.

In particular, during sequencing, the obtained transcriptome library (FIG. 5) and spatial tag library (FIG. 6) are sequenced separately. The sequencing can be performed using an Illumina sequencer or a BGI sequencer, and any sequencing platform compatible with the sequencing adapter. Further, a person of ordinary skill in the art can modify the primer sequence in front of the spatial tag according to the requirements of different sequencing platforms to apply to different sequencing platforms, without affecting the technical protection scope of the present disclosure.

In particular, during data analysis, the single-cell transcriptome data generated from sequencing is analyzed according to standard analytical procedures. Firstly, the estimated number of cells and corresponding cell tags can be obtained based on the number of UMIs detected for all cell tags. Further, the type and physiological state of each cell are determined based on the specific RNA expression corresponding to each cell tag. Secondly, the spatial tag information corresponding to all cell tags is obtained by analyzing the spatial tag library data, each detected cell tag can correspond to at least one spatial tag, and the cell can be mapped to the original position on the chip based on the spatial tag. If the detected cell tag corresponds to multiple spatial tags, the UMIs of all spatial tags need to be counted, sorted, and then deconvolved, to determine the true position.

The sequencing method provided in the present disclosure includes both the technical solutions of the method for preparing a spatial barcode chip and the high-throughput single-cell library construction method, and therefore, has all the beneficial effects of the method for preparing a spatial barcode chip and the high-throughput single-cell library construction method. Details will not be repeated in the present disclosure.

The technical solution in the present disclosure is further described in detail with reference to the specific examples and accompanying drawings. It should be understood that the following examples are used only to explain the present disclosure, but are not used to limit the present disclosure.

### Example 1 Preparation of spatial barcode chip

1.1. Nucleic acid molecular libraries with different spatial tag sequences were prepared, the nucleic acid molecular libraries could be synthesized by a synthesis company or researchers, and the steps of preparing the nucleic acid molecular libraries include:
1.1.1. Single-stranded nucleic acids with the following sequences were synthesized separately:

**Table 1: 3 types of single-stranded nucleic acid molecules**

| **SEQ ID No.** | **Name** | **Sequence** |
|---|---|---|
| **1** | **SK-1** | AATGATACGGCGACCACCGAGATCTACACGTGAC TGGAGTTCAGACGTGTGCTCTTCCGATCTNNNNN NNNNNNNNNNNNNNNNNNNNNNNNNNNTCTTG TGACTACAGCACC |
| **2** | **SK-2** | p-GACTTTCACCAGTCCATGATAAAAAAAAAAAAA AAAAAAAAAAAAAAAAAAAAAATCTCGTATGCCGTC TTCTGCTTG |
| **3** | **SK-2 IR** | **GGTGAAAGTCGGTGCTGTAGT** |

| | | |
|---|---|---|
| P: Phosphate modification SK-21R: A partially complementary sequence of the sequencing primer binding site for decoding; | | |

1.1.2. 3 types of single-stranded nucleic acids according to the following ratios were each added into a PCR tube:

**Table 2: Reaction system of the 3 types of single-stranded nucleic acids**

| Reagent | 1 00 µL system |
|---|---|
| 5×ligation buffer | 20 µL |
| T4 DNA ligase | 4 µL |
| 100umSK-1 | 5 µL |
| 100umSK-2 | 5 µL |
| 100umSK-21R | 5 µL |
| ddH₂O | Diluted to 100 µL |

1.1.3.

**Table 3: PCR reaction conditions of the 3 types of single-stranded nucleic acids**

| Temperature | Time |
|---|---|
| 20°C | 3 h |
| 65°C | 10min |
| 4°C | Hold |

1.1.4. After the reaction, 10% denaturing PAGE gel electrophoresis was performed, and the band around 190bp was cut and recovered.
1.1.5. After recovery, 2 volumes of DNA clean beads were added for purification, and eluted with 50µL of double-distilled water.
1.1.6. The specific sequence after ligation is shown as SEQ ID NO.4: 
AATGATACGGCGACCACCGAGATCTACACGTGACTGGAGTTCAGACGTGTGCT
CTTCCGATCTNNNNNNNNNNNNNNNNNNNNNNNNNNNNNNNNTCTTGTGACTA
CAGCACCGACTTTCACCAGTCCATGATAAAAAAAAAAAAAAAAAAAAAAAAAAAA
AAATCTCGTATGCCGTCTTCTGCTTG
The P5 sequence is shown as SEQ ID NO.5: AATGATACGGCGACCACCGAGATCTACAC
The sequencing adapter sequence is shown as SEQ ID NO.6:
   GTGACTGGAGTTCAGACGTGTGCTCTTCCGATCT
Spatial tag: NNNNNNNNNNNNNNNNNNNNNNNNNNNNNNNN
The sequencing primer binding site for decoding is shown as SEQ ID NO.7: TCTTGTGACTACAGCACCGACTTTCACCAGTCCATGAT
The P7' sequence (complementary sequence of P7) is shown as SEQ ID NO.8:
   ATCTCGTATGCCGTCTTCTGCTTG

1.1.7. Generation of spatial barcode chip and decoding of nucleic acid sequence:
The prepared nucleic acid molecular libraries were added to GenoLab M chip for bridge amplification using Genemind nanoscale platform, then the P7 sequence on the chip was removed by Fpg enzyme, the P5 sequence molecule clusters were retained, and the spatial tag sequence was sequenced and decoded, where the binding region of the sequencing primer was the sequencing primer binding site for decoding; and then cyclic sequencing reactions were performed on the sequencing chip, the spatial tags were decoded, where each cluster had spatial information encoded by a known nucleic acid base sequence; and NaOH denaturation was performed after the sequencing was completed, the sequencing strand was removed, and only the original template strand of the spatial tag sequence molecule on the chip was retained.

The chip was cut into a size of 5.5 mm*15 mm using a laser, where one Flowcell sample could be cut into 4 chips, with the surface having the nucleic acid sequence facing upwards; and the chip was fixed onto a slide using transparent double-sided tape, the chip was cleaned and then the chip was stored in a sealed chip box at 4°C for later use, to obtain the spatial barcode chip.

### Example 2 Single-cell transcriptome library construction for formaldehyde-fixed tissue with spatial sequencing chip

2.1. OCT-embedded frozen mouse liver tissue was taken, and the tissue sample was sliced using a freezing microtome.
2.2. Slices were made at 30um thickness and attached to the spatial barcode chip prepared in Example 1, baked at 37°C for 2 minutes.
2.3. 200 µL of a nucleus extraction solution containing 0.1% NP40 was used on ice for 4 minutes to permeabilize the tissue cell membrane.
2.4. 200 µL of USER enzyme reaction solution was prepared according to NEB instruction manual, added dropwise to the tissue, and allowed to react at 37°C for 30 minutes to release the spatial tag primers and bind them to the tissue cell nuclei nearby to achieve spatial tagging, and environmental humidity was controlled to prevent the reaction solution from drying.
2.5. The chip was gently washed twice with 200 µL of 1× PBST to remove unbound free primers.
2.6. The cell nuclei containing spatial tag primers were fixed with 200 µL of 4% paraformaldehyde for 10 minutes, the fixative solution contained 0.5 µL of Qubit ssDNA dye, and during fixation, fluorescent photographs were taken for the distribution of the tissue cell nuclei.
2.7. The cell nuclei containing spatial tag primers were gently recovered from the spatial chip with 200 µL of 1 × PBST.
2.8. The cell nuclei obtained from the dissociation in step 2.7 were reversely transcribed in a water-in-oil reagent using the SeekOne^{®} DD Single Cell 3' Transcriptome kit, and the cDNA product was purified by oil breaking.
2.9. Demulsification and decrosslinking
   2.9.1. After the water-in-oil reaction was completed, 100 µL of Demulsion Agent reagent was added into the PCR tube, and the resulting mixture was left at room temperature for 2 minutes and briefly centrifuged.
   2.9.2. The transparent oil phase was slowly aspirated from the bottom of the PCR tube and discarded, the upper aqueous phase was not aspirated, and at this time, the reversely transcribed fixed nuclei were present in the upper aqueous phase.
   2.9.3. Proteinase K and 2% (v/v) SDS were added into the tube, and decrosslinking reaction was performed at 55°C for 1 hour.
   2.9.4. After the reaction ended, 1.8 volumes of DNA clean beads were added into the tube for purification, and 30.5 µL of double-distilled water was added for elution.
   2.9.5. 30 µL of the supernatant was transferred to a new 0.2 mL centrifuge tube.
2.10. Template conversion

The template conversion system was prepared using the components in the SeekOne^{®} DD Single Cell 3' Transcriptome kit according to the following formula:

**Table 4: Template conversion system**

| Reagent | Volume/Sample |
|---|---|
| RT Buffer | 26.6µL |
| Indicator | 8.0 µL |
| RT Enzyme | 5.2 µL |
| ddH2O | 6.6 µL |
| TSO | µL 2.0 |
| Reducing Buffer | 1.6 µL |
| Total | 50 µL |

2.10.1. The prepared template conversion system was added into the purified product from step 2.9.5, and allowed to react for 90 minutes at 42°C and then inactivated at 85°C for 5 minutes.
2.10.2. 1.8 volumes of DNA clean beads were added for purification, and 22.6 µL of double-distilled water was used for elution.
2.11. The amplification mix was prepared according to the table below.

The amplification system was added into 23 µL of cDNA product purified according to the instruction for the SeekOne^{®} DD Single Cell 3' Transcriptome kit, mixed by pipetting up and down 10 times, and centrifuged briefly; PCR was then performed according to Step 2-2 of the instruction for the SeekOne^{®} DD Single Cell 3' Transcriptome kit. Note that additional spatial primers at fixed concentrations need to be added in addition to the standard reagent system.

**Table 5: Amplification mix**

| Component | Volume |
|---|---|
| 2 × PCR Master Mix | 25 uL |
| cDNA Primers | 2 uL |
| Spatial primer (2.5mM) | 0.4 uL |
| Primer sequence: | |
| GTGACTGGAGTTCAGACGTGTGC | |
| Total | 27.4 uL |

### 2.12. PCR product purification

30 µL (0.6×) of DNA sorting magnetic beads were pipetted and added into the cDNA amplification product, mixed by pipetting up and down or oscillating 10 times, and then centrifuged briefly. The mixed product was left at room temperature for 5 minutes, then placed on a magnetic stand for absorption until the solution was clear, and all the supernatant was transferred to a new 0.2 mL PCR tube and stored at room temperature. A. The product obtained by purification of the magnetic beads after removal of the supernatant was used for 3' transcriptome library construction; and B. 40 µL of the supernatant was transferred to a new 0.2 mL PCR tube, and the product obtained from purification of the supernatant was used for sample tag library construction.
2.12-A. Purification of magnetic beads (for 3' transcriptome library construction)
   2.12-A.1. The resulting product was kept on the magnetic stand, 200 µL of 80% ethanol was added, and the supernatant was carefully removed after about 30 seconds. The step was repeated once.
   2.12-A.2. The resulting product was briefly centrifuged, and all residual supernatant was removed using a 10 µL pipette.
   2.12-A.3. The resulting product was left at room temperature to allow the ethanol to completely evaporate (the magnetic beads appeared matte, about 3 minutes to 5 minutes), 40.5 µL of Nuclease-free Water was added to fully suspend the magnetic beads, and the resulting product was left at room temperature for 2 minutes.
   2.12-A.4. The resulting product was placed on the magnetic stand for absorption until the solution was clear, and 40 µL of the supernatant was transferred to a new 0.2 mL PCR tube.
2.12-B. Purification of supernatant (for spatial tag library construction)
   2.12-B.1. The DNA sorting magnetic beads were oscillated and mixed evenly, 35 µL (2×) of DNA sorting magnetic beads were pipetted and added into the supernatant, mixed by pipetting up and down or oscillating 10 times, and then centrifuged briefly.
   2.12-B.2. The mixed product was left at room temperature for 5 minutes, then placed on a magnetic stand for absorption until the solution was clear, and the supernatant was removed.
   2.12-B.3. The resulting product was kept on the magnetic stand, 200 µL of 80% ethanol was added, and the supernatant was carefully removed after about 30 seconds. The step was repeated once.
   2.12-B.4. The resulting product was briefly centrifuged, and all residual supernatant was removed using a 10 µL pipette.
   2.12-B.5. The resulting product was left at room temperature to allow the ethanol to completely evaporate (the magnetic beads appeared matte, about 3 minutes to 5 minutes), 20.5 µL of Nuclease-free Water was added to fully suspend the magnetic beads, and the resulting product was left at room temperature for 2 minutes.
   2.12-B.6. The resulting product was placed on the magnetic stand until the solution was clear, and 20 µL of the supernatant was transferred to a new 0.2 mL PCR tube.

### 2.13. Construction of 3' transcriptome library

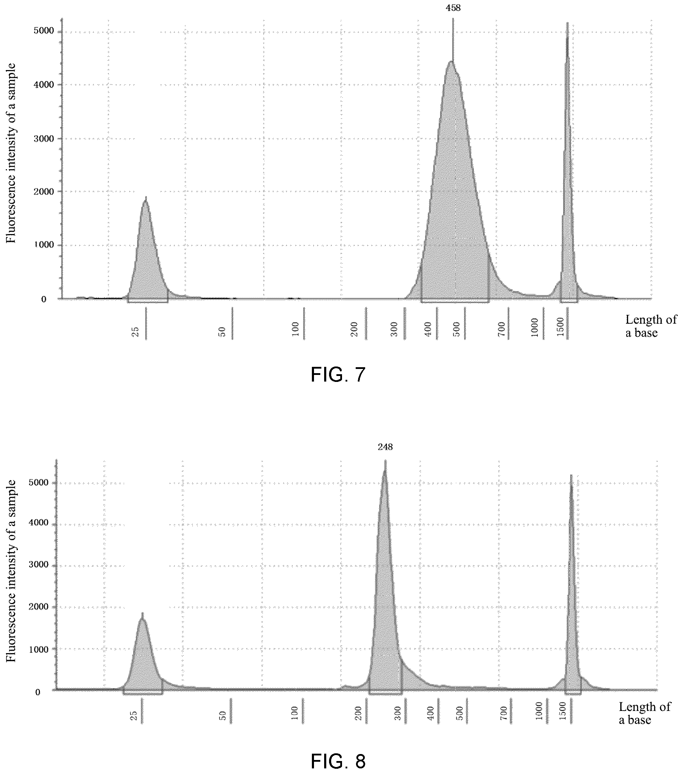

The purified cDNA product from step 2.12-A was used to construct the 3' transcriptome library according to the library construction in Step 3 of the instruction for the SeekOne^{®} DD Single Cell 3' Transcriptome kit. The final library is shown in FIG. 7: the main peak of the library fragment was 458 bp, with no smearing bands.

### 2.14. Construction of sample tag library

### 2.14.1. Amplification of sample tag library

The spatial tag library was constructed using the purified DNA product from Step 2.12-B. A sample of Index PCR Mix was prepared according to the following table, and about 1 ng to 2 ng of the purified DNA product from Step 4-2B was added into the amplification system, and the mixture was mixed by pipetting up and down 10 times, and centrifuged briefly.

**Table 6 Sample of indexPCR Mix**

| Component | Volume |
|---|---|
| 2 ×PCR Master Mix | 25 µL |
| N5 | 1 µL |
| N7 | 1 µL |
| ddH2O | To 50 µL |

| | |
|---|---|
| Note: The sample tag library and its 3' transcriptome library cannot use the same set of N5 and N7. | |

2.14.2. The PCR program was set and run according to the table below.

**Table 7: PCR program parameters**

| Temperature | Time | Number of amplification cycles |
|---|---|---|
| 98°C | 3 min | |
| 98°C | 10 sec | 13 cycles |
| 63°C | 15 sec | |
| 72°C | 3 min | |
| 72°C | 5 min | |
| 4°C | hold | |

### 2.14.3. Purification of sample tag library

60 µL (1.2×) of DNA sorting magnetic beads were pipetted and added into the PCR product, mixed by pipetting up and down or oscillating 10 times, and then centrifuged briefly.

The mixed product was left at room temperature for 5 minutes, then placed on a magnetic stand for absorption until the solution was clear, and the supernatant was removed.

The resulting product was kept on the magnetic stand, 200 µL of 80% ethanol was added, and the supernatant was carefully removed after about 30 seconds. The step was repeated once.

The resulting product was briefly centrifuged, and all residual supernatant was removed using a 10 µL pipette.

The resulting product was left at room temperature to allow the ethanol to completely evaporate (the magnetic beads appeard matte, about 3 minutes to 5 minutes), 30.5 µL of Nuclease-free Water was added to fully suspend the magnetic beads, and the resulting product was left at room temperature for 2 minutes.

The resulting product was placed on the magnetic stand for absorption until the solution was clear, and 30 µL of the supernatant was transferred to a new 0.2 mL PCR tube.

### 2.14.4. Quality control of sample tag library

As shown in FIG. 8, the main peak size of the qualified library was from 200 bp to 350 bp (Agilent 4200 TapeStation) and the library concentration was >1 ng/µL (measured by Qubit 4.0).

### 2.14.5. High-throughput sequencing

The transcriptome library and the sample tag library were mixed in a ratio of 4:1, and high-throughput sequencing was performed using the Illumina sequencing platform and BGI sequencing platform, with a sequencing volume of ≥50000 reads/cell.

The above are only preferred examples of the present disclosure and are not intended to limit the scope of the present disclosure. Various modifications and changes may be made to the present disclosure by a person skilled in the art. Any modifications, equivalent replacements, improvements, etc., made within the spirit and principles of the present disclosure should be included within the protection scope of the invention.

## Claims

1. A method for preparing a spatial barcode chip, **characterized by** comprising steps of:
S10: providing a chip;
S20: binding multiple nucleic acid molecules from a nucleic acid molecule library to cleavable sequences of the chip, respectively, and performing amplification to obtain multiple nucleic acid molecule clusters, wherein, each of the nucleic acid molecules includes a spatial tag sequence and a library construction binding sequence, and a sequencing primer binding site for decoding between the spatial tag sequence and the library construction binding sequence, the spatial tag sequence of each of the nucleic acid molecules is different, and the nucleic acid molecule further includes a chip binding sequence which binds to a corresponding cleavable sequence on the chip; and
S30: sequencing and decoding the nucleic acid molecule clusters to obtain the spatial barcode chip.

2. The method for preparing a spatial barcode chip of claim 1, **characterized in that**,
the library construction binding sequence comprises one base or a combination of at least two bases; and/or
the cleavable sequence of the chip comprises any one of a disulfide-modified sequence, a dU-modified sequence, a dl-modified sequence, an AP site-modified sequence, an i8oxodG-modified sequence, a photolabile group-modified sequence, and a restriction enzyme recognition sequence.

3. The method for preparing a spatial barcode chip of claim 1, **characterized in that**,
the chip is ligated to a primer for in situ amplification, and the primer contains a cleavable sequence; and
optionally, the primer further contains a modified group with a ligation function, and a dNTP derivative that provides dNTP in the amplification and has a chemical ligation function.

4. The method for preparing a spatial barcode chip of claim 1, **characterized in that**, in step S20, the nucleic acid molecule library is prepared by steps of:
S201: providing various single-stranded nucleic acid molecules; and
S202: subjecting the various single-stranded nucleic acid molecules to a ligation reaction to obtain the nucleic acid molecule library.

5. The method for preparing a spatial barcode chip of claim 4, **characterized in that**, in step S201, the various single-stranded nucleic acid molecules include a first nucleic acid molecule, a second nucleic acid molecule, and a third nucleic acid molecule;
wherein a sequence of the first nucleic acid molecule is shown as SEQ ID NO.1;
a sequence of the second nucleic acid molecule is shown as SEQ ID NO.2; and/or
a sequence of the third nucleic acid molecule is shown as SEQ ID NO.3.

6. The method for preparing a spatial barcode chip of claim 4, **characterized in that**, in step S202, a sequence of the nucleic acid molecule is shown as SEQ ID NO.4.

7. The method for preparing a spatial barcode chip of claim 1, **characterized in that**,
the library construction binding sequence is 5 bp to 100 bp;
a density of each of the nucleic acid molecule clusters is 0.1 um² to 2 um²; and/or
the spatial tag sequence is 15 bp to 40 bp.

8. A high-throughput single-cell library construction method, **characterized by** comprising steps of:
S40: providing a spatial barcode chip prepared by the method for preparing a spatial barcode chip of any one of claims 1 to 7;
S50: contacting the spatial barcode chip with a slice of a tissue to be tested and performing dissociation to prepare a single-cell nuclei suspension;
S60: constructing a library for the single-cell nuclei suspension using a high-throughput single-cell library construction and sequencing platform to obtain a transcriptome library and a spatial tag library; and
S70: sequencing the transcriptome library and the spatial tag library to obtain single-cell library data containing spatial information of cells in tissue.

9. The high-throughput single-cell library construction method of claim 8, **characterized in that**, step S50 comprises a step of:
S501: contacting the spatial barcode chip with the slice of the tissue to be tested, then baking the slice, extracting nuclei, releasing nucleic acid molecules with spatial information, and performing fixation and dissociation to obtain the single-cell nuclei suspension.

10. The high-throughput single-cell library construction method of claim 9, **characterized in that**,
a reagent for extracting nuclei in step S501 comprises at least one of NP40, Chaps, Tween20, TritonX-100, SDS, and deoxycholic acid; and/or
a reagent for the fixation comprises at least one of an aldehyde fixative solution, an acid fixative solution, an alcohol fixative solution, and a DSP crosslinker.

11. A sequencing method, **characterized by** comprising steps of:
constructing a single-cell library using the high-throughput single-cell library construction method of any one of claims 8 to 10 to obtain a transcriptome library and a spatial tag library;
sequencing the transcriptome library and the spatial tag library to obtain single-cell transcriptome data and spatial tag library data; and
mapping spatial information and genetic information of nucleic acid molecules to the tissue to be tested to obtain spatial information and genetic information of single cells in the tissue to be tested.
